Europäisches Patentamt

European Patent Office

Office européen des brevets

(11) Veröffentlichungsnummer: **0 224 868 B1**

(12) **EUROPÄISCHE PATENTSCHRIFT**

(45) Veröffentlichungstag der Patentschrift: 28.08.91

(51) Int. Cl.⁵: **A61K 31/71**, A61K 7/48, A61K 47/00

(21) Anmeldenummer: 86116439.0

(22) Anmeldetag: 26.11.86

Die Akte enthält technische Angaben, die nach dem Eingang der Anmeldung eingereicht wurden und die nicht in dieser Patentschrift enthalten sind.

(54) **Primycin enthaltendes kolloidales Grundgel, Verfahren zu dessen Herstellung, dieses Grundgel enthaltende pharmazeutische und pharmako-kosmetische Kompositionen.**

(30) Priorität: 27.11.85 HU 452685
14.11.86 HU 468986

(43) Veröffentlichungstag der Anmeldung:
10.06.87 Patentblatt 87/24

(45) Bekanntmachung des Hinweises auf die Patenterteilung:
28.08.91 Patentblatt 91/35

(84) Benannte Vertragsstaaten:
AT BE CH DE ES FR GB GR IT LI NL SE

(56) Entgegenhaltungen:
DE-A- 2 615 140
DE-A- 3 104 282
US-A- 3 957 994
US-A- 4 086 332

(73) Patentinhaber: CHINOIN Gyogyszer és Vegyészeti Termékek Gyára RT.
To utca 1-5
H-1045 Budapest IV(HU)

(72) Erfinder: Szentmikl si, Péter, Dr.
Rudas L.u. 75
H-1064 Budapest(HU)
Erfinder: Szüts, Tamás, Dr.
Csalogány u. 53
H-1027 Budapest(HU)
Erfinder: Nemes, J zsef
Csaktornya u. 9
H-1142 Budapest(HU)
Erfinder: Lengyel, J zsef, Dr.
Tel u. 46
H-1043 Budapest(HU)
Erfinder: Marton, Jenö, Dr.
Szél u. 19
H-1035 Budapest(HU)
Erfinder: Erzsébet geb. Vajas, Babos
Vezér u. 140/b
H-1148 Budapest(HU)

Erfinder: **Schreiner, geb. Kovats, Enikö, Dr.**
**Belgrad-rkp. 18**
**H-1056 Budapest(HU)**
Erfinder: **Sárközi, Péter, Dr.**
**Várfok u. 10**
**H-1012 Budapest(HU)**

(74) Vertreter: **Bartsch, Elisabeth, Dr.**
**Patentanwälte Lotterhos & Partner Lichten-**
**steinstrasse 3**
**W-6000 Frankfurt am Main 1(DE)**

**Beschreibung**

Die Erfindung betrifft, wie in den Patentansprüchen näher gekennzeichnet, ein Primycin enthaltendes kolloidales Grundgel, ein Verfahren zu dessen Herstellung, dieses Grundgel enthaltende pharmazeutische und pharmako-kosmetische Kompositionen und ein Verfahren zur Herstellung derselben.

Primycin ist ein makrolides Antibiotikum, das in der Fachliteratur (J. Chem. Soc. Perkin I, Seite 816 (1974)) durch eine einzige Formel charakterisiert worden ist, und zwar als [5-(18-[$\alpha$-D-Arabinofuranosyloxy]-2-butyl-3,7,11,15,19,21,23,25,27-nonahydroxy-4,16,32,34-tetramethyl-1-oxo-oxacyclo-hexatriakonta-16,32-dien-35-yl)-4-hydroxyhexyl]-guanidinium-sulfat. Obwohl in der Literatur Primycin durch eine einzige Formel charakterisiert wird, handelt es sich um einen aus mehreren Komponenten bestehenden Antibiotikumkomplex (HU-A 195 514 und 196 425).

Primycin ist ein von dem Fungistamm Thermopolyspora galeriensis produziertes natürliches Antibiotikum, dessen Herstellung auf fermentativem Wege in der HU-A- 153 593 beschrieben ist. Primycin ist ein Antibiotikum mit einem breiten Wirkungsspektrum, es ist hauptsächlich gegenüber gram-positiven Bakterien wirksam und sogar gegenüber polyresistenten humanpathogenen Stämmen aktiv. Eine Resistenz gegen Primycin hat sich bisher noch nicht entwickelt. Mit anderen Antibiotika zeigt Primycin einen Synergismus (HU-A- 158 241). Primycin kann in der Dermatologie, Chirurgie, Ophthalmologie, Urologie, Gynäkologie, Phthisiologie sowie bei der Behandlung von Haushaltsverletzungen und Brandwunden mit Erfolg eingesetzt werden. Primycin hat sich bei der Behandlung von Akne vulgaris in der Dermatologie als besonders wirksam erwiesen.

Die therapeutische Anwendung von Primycin wird durch seine äusserst schlechte Lösbarkeit sehr erschwert, es ist relativ schwer, Arzneimittelformen herzustellen, aus denen der Wirkstoff leicht zugänglich ist. Diese Schwierigkeiten werden nach dem Vorschlag der HU-A- 173 708 dadurch umgangen, daß in einem wässrig-alkoholischen Medium ein 0,2 % Primycin enthaltendes stabiles Heterokolloid gebildet wird, das jedoch zwei grundsätzliche Nachteile aufweist: einerseits ist der Wirkstoff nach Verdampfung des Lösungsmittels biologisch nicht mehr zugänglich und andererseits beträgt die Obergrenze der erreichbaren Konzentration nur 0,2 %. Dies schränkt die Anwendbarkeit der Komposition ein, weil die zur Behandlung von Akne vulgaris verwendeten bekannten Antibiotikum enthaltenden Präparate einen Wirkstoffgehalt von 1-2 % aufweisen.

Das Ziel der Erfindung war daher, die Entwicklung eines geeigneten Primycin enthaltenden Mittels, aus dem sich pharmazeutische und/oder pharmako-kosmetische Präparate mit vorteilhaften Eigenschaften herstellen lassen. Es wurde überraschender Weise gefunden, daß durch Auf lösen von Primycin in N-Methyl-pyrrolidon-2 (weiterhin NMP) ein Grundgel stabiler Struktur erhalten wird, aus dem verschiedene Primycinpräparate mit einem wesentlich höheren Primycingehalt als dem der bekannten Präparate hergestellt werden können. Dies ermöglicht die Herstellung von beliebigen pharmazeutischen Formen, die Primycin in Konzentrationen bis zu 30 % aufweisen können.

Aus der DE-A-26 15 140 ist es zwar bekannt, verschiedene Arzneistoffe in niedriger Dosierung, u.a. auch Antibiotika oder spezielle Makrolid-Antibiotika,in ein Trägersystem mit einem Gehalt an 2-Pyrrolidin und N-Methyl-2-pyrrolidon gemischt einzuarbeiten mit dem Ziel einer transdermalen Wirksamkeit. Der exemplifizierte Antibiotikagehalt betrug dabei aber nur 05, bzw. 1 %.

Dagegen wurde gefunden, daß 5-30 g Primycin, die in 100 ml N-Methyl-pyrrolidon-2 bei 100 °C gelöst werden, nach dem Abkühlen der so erhaltenen Lösung ein stabiles Gel bilden, das sowohl als solches als auch als Grundlage für beliebige übliche Präparate (z.B. Gele, Salben, Sprays, Lösungen usw.) verwendet werden kann.

Die Erfindung betrifft ein Primycin enthaltendes kolloidales Grundgel, das einen Gehalt an 5-30 % Primycin und 95-70 % N-Methyl-pyrrolidon-2, vorzugsweise an 10-20 % Primycin und an 90-80 % N-Methyl-pyrrolidon-2 aufweist.

Die Erfindung betrifft auch ein Verfahren zur Herstellung eines Primycin enthaltenden kolloidalen Grundgels, das dadurch gekennzeichnet ist, daß man 5-30 % Primycin in 95-70 % N-Methyl-pyrrolidon-2 bei einer Temperatur von 50-150 °C, gewünschtenfalls unter Rühren, löst und danach die Lösung auf Raumtemperatur abkühlt.

Nach einer vorteilhaften Ausführungsform dieses Verfahrens werden 10-20 % Primycin in 90-80 % N-Methyl-pyrrolidon-2 gelöst. Das Verfahren wird vorzugsweise bei einer Temperatur von 70-100 °C durchgeführt.

Die Erfindung betrifft weiterhin ein antibakterielles Präparat, insbesondere zur Behandlung von Akne vulgaris, das als Wirkstoff 0,1-100 % eines Primycin enthaltenden kolloidalen Grundgels, gewünschtenfalls weitere pharmazeutische Wirkstoffe und 99,9-0 % übliche pharmazeutische Füllstoffe, Verdünnungsmittel und Hilfsstoffe enthält.

Die Erfindung betrifft ferner ein Kombinationspräparat, das als Wirkstoff 1-60 % eines Primycin enthaltenden kolloidalen Grundgels und in einer Menge von 0,1-40 % einen oder mehrere weitere pharmazeutische Wirkstoffe, insbesondere Antibiotika, chemotherapeutische Mittel, fungistatische oder fungizide Mittel, steroidartige oder nicht-steroidartige antiphlogistische Mittel, die Epithelbildung fördernde Mittel, lokalanästhetische Mittel und/oder Vitamine enthält.

Zur Herstellung dieser antibakteriellen pharmazeutischen und/oder pharmako-kosmetischen Präparate wird zweckmässig so vorgegangen, daß man ein Primycin und gewünschtenfalls weitere antibakterielle Wirkstoffe - insbesondere Antibiotika - enthaltendes kolloidales Grundgel unter Anwendung von üblichen Trägern, Füllstoffen und/oder Hilfsstoffen in die Form von pharmazeutischen und/oder pharmako-kosmetischen Präparaten überführt.

Die Präparate können in die üblichen Formen - wie Gele, Salben, Lösungen, z.B. zum Aufbringen mit dem Pinsel, Sprays, Streupuder oder andere zur lokalen Anwendung geeigneten Formen oder durch Aufbringen auf sterilen Mull in Form von Verbänden oder Pflastern - gebracht werden.

Das N-Methyl-pyrrolidon-2 ist ein in kosmetischen Präparaten oft verwendetes Lösungsmittel, bei dessen vorteilhaften Eigenschaften -d.h. die geringe Toxizität und die unbegrenzte Mischbarkeit mit Wasser, organischen Lösungsmitteln und Fetten - genutzt wird. Aufgrund dieser Eigenschaften ist N-Methyl-pyrrolidon-2 zur Herstellung von Primycin enthaltenden pharmazeutischen Präparaten besonders geeignet, weil bei der Formulierung dieses Gels mit Hilfe von irgendwelchen Lösungsmitteln oder Hilfsmitteln, sich die ursprüngliche Gelstruktur nach Verdampfen oder Absorption des Lösüngsmittels während der Anwendung wieder herstellt. Dies fördert die Primycinpenetration zwischen den biologischen wässrigen und Lipidphasen.

Es wurde die spontane Gelbildung sowohl mit verschiedenen Lösungsmitteln wie auch mit verschiedenen pharmazeutischen Wirkstoffen untersucht, wobei die folgenden Lösungsmittel Methanol, Äthanol, Isopropanol, n-Butanol, Isobutanol, Dichlormethan, Chloroform, Tetrachlorkohlenstoff, Äthylacetat, Acetonitril, n-Hexan, Benzin, Benzol, Toluol, Petroläther, Äther, Dimethylformamid, Dimethylsulfoxyd, Isopropylmyristat verwendet wurden. Hierbei stellte sich heraus, daß keines der untersuchten Lösungsmittel zur Bildung eines stabilen Gels mit Primycin fähig ist.

Andere Lösungsmittel (z.B. Äthanolamin, Diäthanolamin, Triäthanolamin, Dimethylformamid, Dimethylsulfoxyd) bilden zwar ein Gel mit Primycin, im untersuchten Konzentrationsbereich von 5-30 % wurde jedoch weder warm noch kalt ein reines Gel gebildet, so daß diese Lösungsmittel für Zwecke der Arzneimittelformulierung ungeeignet sind.

Die Gelbildung zwischen anderen pharmazeutischen Wirkstoffen und N-Methyl-pyrrolidon-2 als Lösungsmittel wurde ebenfalls untersucht, wobei die Wirkstoffe: Gentamycin, Doxycyclin, Oxolinsäure, Nalidixinsäure, Drotaverin, Theophyllin, Acetylsalicylsäure, Papaverin, Indometacin eingesetzt wurden. Auch hier wurde in keinem der Fälle ein stabiles und durchsichtiges Gel gebildet.

Eine spontane Gelbildung, die zu einem stabilen durchsichtigen Gel führt, ist nur das Ergebnis der Zwischenwirkung von Primycin und N-Methyl-pyrrolidon-2.

Die Möglichkeit, zwei Wirkstoffe - u.a. von Primycin verschiedene Wirkstoffe - enthaltende gemischte Gele zu bilden, wurde z.B. unter Verwendung von Gentamycin, Doxycyclin, Oxytetracyclin, Chloramphenicol, usw. studiert. Es wurde gefunden, daß keine Gelbildung erfolgt, wenn man der Lösung von Primycin mit N-Methyl-pyrrolidon-2 die getesteten Antibiotika in fester Form zugibt. Wird dagegen Doxycyclin oder Gentamycin im Wasser gelöst und die erhaltene Lösung der Lösung von Primycin mit N-Methyl-pyrrolidon-2 zugefügt, beginnt die Gelbildung und es werden zwei Wirkstoffe (d.h. Primycin und Doxycyclin bzw. Primycin und Gentamycin) enthaltende Hydrogele erhalten.

Die spezifische Wechselwirkung zwischen Primycin und N-Methyl-pyrrolidon-2 kann einerseits der Temperaturabhängigkeit der Lösbarkeit (zwischen 0 und 100 °C mehr als zwei Grössenordnungen) und andererseits der multikomponenten Zusammensetzung des Primycins zugeschrieben werden.

Es wurde die Stabilität sowie die Änderung der chemischen bzw. mikrobiologischen Aktivität des erfindungsgemäßen Primycin-Grundgels geprüft, wobei das verwendete, 20 % Primycin enthaltende Grundgel 5 Stunden bei 100 °C stehen gelassen bzw. einen Monat bei Raumtemperatur und einen Monat bei +5 °C gelagert wurde. Hierbei stellte sich heraus, daß sich während der Lagerung weder die Struktur noch die chemische Zusammensetzung bzw. die mikrobiologische Aktivität des Gels geändert hatte. Dies bedeutet, daß durch die Gelbildung die für die pharmazeutische Anwendung wichtigen Eigenschaften des Primycins nicht verändert werden.

Die so gebildete stabile Gelform kann als Grundlage zur Herstellung von Primycin enthaltenden therapeutischen oder veterinär-medizinischen Präparaten verwendet werden. Die so hergestellten pharmazeutischen Präparate können entweder nur Primycin als Wirkstoff enthalten (Monotherapie) oder mit anderen Wirkstoffen (z.B. Antibiotika, chemotherapeutischen Mitteln, steroidartigen oder nicht-steroidartigen antiphlogistischen Mitteln, die Epithelbildung fördernden Mitteln, Vitaminen, Lokalanästhetica usw.) kombiniert werden.

Weitere Einzelheiten der vorliegenden Erfindung sind den nachstehenden Beispielen zu entnehmen.

Beispiel 1

Grundgel mit einem Primycingehalt von 20 %
20,0 g Primycin werden in 80,0 g N-Methyl-pyrrolidin-2 bei 100°C auf einem Wasserbad gelöst. Die Gelbildung beginnt bereits während des Auflösens und wird nach dem Abkühlen auf Raumtemperatur vollständig. Das so erhaltene 20 %ige Grundgel wird zur Herstellung der verschiedenen pharmazeutischen Präparate verwendet.

Beispiel 2

0,5 % Primycin enthaltendes wässriges Gel
9,75 g Avicel® RC 581 (FMC Corporation, Philadelphia, USA) werden mit 87,75 g destilliertem Wasser in kleinen Portionen bei Raumtemperatur unter ständigem Rühren homogenisiert. Das so erhaltene Gel wird mit 2,5 g 20 %igem Grundgel weiter homogenisiert.

Beispiel 3

1,0 % Primycin enthaltendes wässriges Gel
9,50 g Avicel® RC 581 (FMC Corporation, Philadelphia, USA) werden mit 85,5 g destilliertem Wasser in kleinen Portionen bei Raumtemperatur unter ständigem Rühren homogenisiert. Das erhaltene Gel wird mit 5,0 g 20 %igem Grundgel weiter homogenisiert.

Beispiel 4

1,0 % Primycin enthaltendes alkoholisches Gel
5,0 g 20 %iges Grundgel werden in 10,0 g 96 %igem Alkohol unter leichtem Erwärmen gelöst.
8,5 g Avicel® RC 581 (FMC Corporation, Philadelphia, USA) werden mit 76,5 g destilliertem Wasser in kleinen Portionen bei Raumtemperatur unter ständigem Rühren homogenisiert. Das so erhaltene Gel wird nach weiterem Homogenisieren mit der Primycin enthaltenden alkoholischen Lösung vermischt.

Beispiel 5

2,0 % Primycin enthaltendes wässriges Gel
9,00 g Ultraamylopektin (SERVA Feinbiochemie, Heidelberg, BRD) werden mit 81,0 g destilliertem Wasser in kleinen Portionen bei Raumtemperatur unter ständigem Rühren homogenisiert. Das entstandene Gel wird mit 10,0 g 20 %igem Grundgel homogenisiert.

Beispiel 6

10,0 % Primycin enthaltendes wässriges Gel
5,00 g Avicel® RC 581 (FMC Corporation, Philadelphia, USA) werden mit 45,0 g destilliertem Wasser in kleinen Portionen bei Raumtemperatur unter ständigem Rühren homogenisiert. Das entstandene Gel wird mit 50 g 20 %igem Grundgel weiter homogenisiert.

Beispiel 7

10 % Primycin enthaltendes alkoholisches Gel
50,0 g eines 20 %igen Grundgels werden mit 50,0 g 96 %igem Alkohol homogenisiert.

Beispiel 8

0,5 % Primycin enthaltende Carbowax-Salbe
44,50 g Carbowax® 400 (Polyoxaethenum 400, USNF), 15,10 g Carbowax® 1540 (Polyoxaethenum 1540, USNF), 21,45 g Carbowax® 4000 (Polyoxaethenum 4000, USNF), 9,75 g Glycerin, 1,85 g Cetylstearylalkohol und 4,85 g Sorbit werden auf einem Wasserbad zusammengeschmolzen.

Die so erhaltene geschmolzene Salbengrundlage wird in kleinen Portionen unter ständigem Rühren mit 2,5 g eines 20 %igen Grundgels homogenisiert.

Beispiel 9

0,5 % Primycin enthaltende alkoholische Salbe

2,5 g eines 20 %igen Grundgels werden in 20,0 g 96 %igem Alkohol unter leichtem Erwärmen gelöst.

35,25 g Carbowax® 400, 12,00 g Carbowax® 1540, 17,05 g Carbowax® 4000, 7,75 g Glycerin, 1,55 g Cetylstearylalkohol und 3,80 g Sorbit werden auf einem Wasserbad zusammengeschmolzen.

Die so erhaltene geschmolzene Salbengrundlage wird in kleinen Portionen unter ständigem Rühren mit der alkoholischen Wirkstofflösung vermischt. Die Salbe wird mit Carbowax 400 auf 100,00 g ergänzt und unter Rühren abgekühlt.

Beispiel 10

2 % Primycin enthaltende Carbowax-Salbe

38,60 g Carbowax® 400, 13,20 g Carbowax® 1540, 18,70 g Carbowax® 4000, 8,5 g Glycerin, 1,80 g Cetylstearylalkohol, 4,20 g Sorbit und 5,0 g Lactyl werden auf einem Wasserbad zusammengeschmolzen.

Die so erhaltene geschmolzene Salbengrundlage wird in kleinen Portionen unter ständigem Rühren mit 10,00 g eines 20 %igen Grundgels homogenisiert.

Beispiel 11

10,0 % Primycin enthaltende alkoholische Salbe

50,0 g eines 20 %igen Grundgels werden mit 20,0 g 96 %igem Alkohol homogenisiert.

13,65 g Carbowax® 400, 4,65 g Carbowax® 1540, 6,60 g Carbowax® 4000, 3,00 g Glycerin, 0,6 g Cetylstearylalkohol und 1,50 g Sorbit werden auf einem Wasserbad zusammengeschmolzen.

Die geschmolzene Salbengrundlage wird in kleinen Portionen unter ständigem Rühren mit dem wirkstoffhaltigen Gel homogenisiert. Die Salbe wird mit Carbowax 400 auf 100,0 g ergänzt und unter Rühren abgekühlt.

Beispiel 12

2 % Primycin enthaltende Lösung

10,0 g eines 20 %igen Grundgels werden in einer Mischung von 40,0 g N-Methyl-pyrrolidon-2 und 50,0 g Alkohol gelöst.

Beispiel 13

Salbe mit einem Gehalt an Primycin und anderen Wirkstoffen

a) Salbe mit einem Gehalt an Primycin, Hydrocortison und Nystatin

| Komponenten | Gehalt (g) |
|---|---|
| Primycin | 20,00 |
| Hydrocortison | 20,00 |
| Nystatin | 20,00 |
| N-Methyl-pyrrolidon-2 (NMP) | 40,00 |

Aus 20,00 g Primycin und 40,0 g N-Methyl-pyrrolidon-2 wird bei 70 °C ein Gel hergestellt, dem bei Raumtemperatur unter ständigem Rühren Nystatin und Hydrocortison zugegeben werden. Die erhaltene, sehr viskose Lösung bildet nach 24 stündigem Stehen ein Gel.

b) Salbe mit einem Gehalt an 1 % Primycin, 1 % Hydrocortison und 1 % (300,000 IE) Nystatin

1 % (300,000 IE) Nystatin

| Komponenten | Menge (g) |
|---|---|
| Primycin - Hydrocortison - Nystatin - NMP-Gel | 5,00 |
| Cetylstearylalkohol | 10,00 |
| Emulgator E 2155 (T. Goldschmidt, Essen, BRD) | 5,00 |
| Destilliertes Wasser | ad 100,00 |

Der Cetylstearylalkohol und der Emulgator E 2155 (Stearylalkohol-Polyglykoläther; Hersteller: T. Goldschmidt, Essen, BRD) werden auf einem Wasserbad unter Rühren bei 60-70 °C zusammengeschmolzen und etwa 80 % des destillierten Wassers derselben Temperatur unter ständigem Rühren in dünnem Strahl zugemischt. Das erhaltene Gel wird mit dem oben hergestellten Primycin - Hydrocortison - Nystatin -NMP - Gel weiter homogenisiert und mit destilliertem Wasser aufgefüllt.

Die so erhaltene Salbe kann zur Behandlung von bakteriellen und fungalen Infektionen eingesetzt werden.

Beispiel 14

Präparat zur Behandlung humaner Mastitis

| Komponenten | Menge, g (für 100,00 g) |
|---|---|
| 20 %iges Primycin-NMP-Grundgel | 10,0 |
| Glycerin | 10,0 |
| Tanninsäure | 5,0 |
| Grundsalbe | 75,0 |
| | 100,0 |

| Komponenten | Menge (g) |
|---|---|
| Natriumlaurylsulfat | 4,0 |
| Destilliertes Wasser | 1,5 |
| Cetylstearylalkohol | 36,0 |
| Paraffinum liquidum | 20,0 |
| Vaselinum album | 40,0 |

Das Natriumlaurylsulfat und das zum Sieden erhitzte Wasser werden mit dem, auf einem Wasserbad geschmolzenen und auf etwa 90 °C erhitzten Cetylstearylalkohol vermischt. Die Flüssigkeit wird auf etwa 110-120 °C erwärmt und so lange kräftig gerührt bis das starke Schäumen aufhört. Die abgekühlte Flüssigkeit wird mit der geschmolzenen Mischung des flüssigen Paraffins und der Vaseline vermischt und bis zum Abkühlen gerührt.

40,0 g der erhaltenen Salbe werden auf einem Wasserbad auf etwa 65-70 °C erwärmt. Die Mischung wird in eine vorerwärmte Reibschale gegeben, worauf ein, aus 1,0 g Solutio conservans und 50,0 g destilliertem Wasser gebildetes und auf etwa 65-70 °C erwärmtes Gemisch unter ständigem Rühren in einem dünnen Strahl zugegeben wird. Die Salbe wird bis zum Erreichen des Gelzustandes stark und danach sorgfältig bis zum Abkühlen gerührt und mit destilliertem Wasser auf 100,0 g ergänzt.

Salbe mit einem Gehalt an einem Primycingrundgel

Eine wie oben angegebene Menge von Tanninsäure wird in einer Reibschale in Glycerin suspendiert und danach mit dem Primycin-NMP-Grundgel homogenisiert. Nach Zugabe der Grundsalbe (Raumtemperatur) werden die gemischten Komponenten vollständig homogenisiert.

Beispiel 15

Creme "für Anwendung während des Tages"

| Komponenten | | Menge, g (für 100,00 g) |
|---|---|---|
| Primycin-NMP-Grundgel (20 %ig) | | 5,00 |
| Cetylalkohol | | 5,00 |
| Emulgator E 2155 (T. Goldschmidt, Essen, BRD) | | 5,00 |
| Spiritus ·· · *96%* | | 5,00 |
| Destilliertes Wasser | ad | 100,00 |

Der Cetylalkohol, der Emulgator E 2155 (Stearylalkohol-Polyglykoläther, Hersteller: T. Goldschmidt, Essen, BRD) und ein grosser Teil des destilierten Wassers werden auf einem Wasserbad bei 60-70 °C unter ständigem Rühren zusammengeschmolzen. Das Primycin-NMP-Grundgel wird unter ständigem Rühren in Portionen zugegeben, dann der konzentrierte Alkohol zugefügt und mit destilliertem Wasser ergänzt.

Beispiel 16

Creme "für die Anwendung in der Nacht"

| Komponenten | | Menge, g (für 100,00 g) |
|---|---|---|
| Primycin-NMP-Grundgel (20 %ig) | | 5,00 |
| Cetylstearylalkohol | | 3,15 |
| Stearinum | | 7,00 |
| Natriumlaurylsulfat | | 0,35 |
| Solutio conservans (Ph.Hg.VI.) | | 0,70 |
| Solitolum | | 2,45 |
| Glycerin | | 30,00 |
| Destilliertes Wasser | ad | 100,00 |

Die Komponenten der Cremegrundlage werden unter ständigem Rühren bei 60-70 °C geschmolzen. Danach gibt man unter Fortsetzung des Rührens das Primycin-Grundgel in Portionen zu und ergänzt die Creme mit destilliertem Wasser.

Beispiel 17

Primycin enthaltender Streupuder

| Komponenten | Menge (g) |
|---|---|
| Primycin-NMP-Grundgel (20 %ig) | 10,00 |
| Zinkoxyd | 40,00 |
| Talk | 50,00 |
| | 100,00 |

Das Zinkoxyd wird mit dem Talk gründlich vermischt und danach durch ein VI. Sieb gesiebt. Die erhaltene Pulvermischung wird mit dem Primycin-NMP-Gel homogenisiert, bei 40 °C 24 Stunden in einem Trockenschrank getrocknet, danach erneut gesiebt (Maschenweite 0,16 mm) und wieder homogenisiert. Es wird eine weisse homogene Pulvermischung erhalten.

Beispiel 18

Primycin enthaltender Streupuder

| Komponenten | Menge (g) |
|---|---|
| Primycin-NMP-Grundgel (20 %ig) | 10,00 |
| N,N-Dimethyl-acetamid | 5,00 |
| Zinkoxyd | 40,00 |
| Talk | 45,00 |

Das Primycin-NMP-Grundgel wird in N,N-Dimethyl-acetamid unter Erwärmen gelöst und die Lösung auf eine, nach Beispiel 15 hergestellte Zinkoxyd-Talk-Pulvermischung gesprüht. Die besprühte Pulvermischung wird in einem Trockenschrank bei 40 °C 24 Stunden stehen gelassen und gesiebt (Maschenweite 0,16 mm). Nach der Homogenisierung wird eine weisse homogene Pulvermischung erhalten.

Beispiel 19

Primycin + Gentamycin enthaltendes gemischtes Grundgel

| Komponenten | Menge, g (für 100,00 g) |
|---|---|
| Primycin-NMP-Grundgel (20 %ig) | 50,00 |
| Gentamycin-sulfat | 20,00 |
| Destilliertes Wasser | 30,00 |

Das Gentamycin-sulfat löst man in destilliertem Wa$ser auf einem Wasserbad von 40 °C und gibt bei dieser Temperatur das Primycin-NMP-Grundgel zu. Es wird eine wasserklare durchsichtige Lösung erhalten, die innerhalb von 24 Stunden zu einem glasartigen durchsichtigen Gel erstarrt. Das so erhaltene gemischte Grundgel enthält 10 % Primycin und 20 % Gentamycin.

Beispiel 20

Primycin und Doxycyclin enthaltendes gemischtes Grundgel

| Komponenten | Menge, g (für 100 g) |
|---|---|
| Primycin-NMP-Grundgel (20 %ig) | 50,00 |
| Doxycyclin | 20,00 |
| Destilliertes Wasser | 30,00 |

Das Doxycyclin löst man in destilliertem Wasser auf einem Wasserbad von 40 °C und gibt bei dieser Temperatur das Primycin-NMP-Grundgel zu. Es wird eine wasserklare durchsichtige Lösung erhalten, die bei Raumtemperatur innerhalb von 24 Stunden zu einem glasartigen durchsichtigen Gel erstarrt.
Das so erhaltene gemischte Grundgel enthält 10 % Primycin und 20 % Doxycyclin.

Beispiel 21

Präparat zur Behandlung von Mastitis in der Veterinärmedizin für die Laktationsperiode

| Komponenten | | Menge, g (für 100,00 g) |
|---|---|---|
| Primycin und Gentamycin enthaltendes gemischtes Grundgel | | 10,00 |
| Emulgator E 2209 (T. Goldschmidt, Essen, BRD) | | 5,00 |
| Procain-hydrochlorid | | 1,60 |
| Destilliertes Wasser | ad | 100,00 |

Der wässrigen Procain-hydrochloridlösung wird zunächst der Emulgator E 2209 (Cetylalkohol-polyglykoläther, Hersteller: T. Goldschmidt, Essen, BRD) und danach unter ständigem Rühren das Primycin-Gentamycin enthaltende gemischte Grundgel zugegeben. Es wird ein zur Behandlung von Mastitis geeignetes pastenartiges Präparat erhalten.

Beispiel 22

Präparat zur Behandlung von Mastitis für die Trockenperiode

| Komponenten | Menge, g (für 100 g) |
|---|---|
| Primycin und Gentamycin enthaltendes gemischtes Grundgel | 10,00 |
| Tagat®R-40 (T. Goldschmidt, Essen, BRD) | 5,00 |
| Ung. simplex | 35,00 |
| Glycerin | 50,00 |

Zusammensetzung von Unguentum simplex (Ph. Hg. VI:)

| Komponenten | Menge, g (für 100 g) |
|---|---|
| Lanalcolum | 6,0 |
| Cetylstearylalkohol | 3,0 |
| Vaselinum album ophthalmicum | 12,0 |
| Vaselinum album | 79,0 |

Zunächst wird das Ung. simplex und das Tagat® R-40 (T. Goldschmidt, Essen, BRD) dem Glycerin zugegeben und danach das Primycin und Gentamycin enthaltende gemischte Grundgel unter ständigem Rühren zugesetzt.

Es wird ein zur Behandlung von Mastitis geeignetes pastenartiges Präparat erhalten.

Beispiel 23

Präparat zur Behandlung von Mastitis für die Trockenperiode

| Komponenten | Menge, g (für 100,00 g) |
|---|---|
| Primycin und Gentamycin enthaltendes gemischtes Grundgel | 10,00 |
| Emulgator E 2209 (T. Goldschmidt, Essen, BRD) | 5,00 |
| Ung. simplex | 30,00 |
| Oleum Helianthi | 55,00 |

Der Emulgator E 2209 (Cetylalkohol-polyglykoläther; Hersteller: T. Goldschmidt, Essen, BRD) wird auf einem Wasserbad geschmolzen und mit Unguentum simplex homogenisiert, danach dem Primycin und Gentamycin enthaltenden Gel zugesetzt und in der Kälte einheitlich homogenisiert. Dann wird das Sonnenblumenöl in Portionen zugegeben.

Beispiel 24

Präparat zur Behandlung von Mastitis für die Trockenperiode

| Komponenten | Menge, g (für 100 g) |
|---|---|
| Primycin und Doxycyclin enthaltendes gemischtes Grundgel | 10,00 |
| Tagat® R-40 (T. Goldschmidt, Essen, BRD) | 5,00 |
| Ung. simplex | 35,00 |
| Glycerin | 50,00 |

Das Unguentum simplex und Tagat R-40 werden dem Glycerin zugegeben, worauf das Primycin und Doxycyclin enthaltende gemischte Grundgel unter ständigem Rühren zugegeben wird.

Es wird ein zur Behandlung von Mastitis geeignetes Präparat erhalten.

**Patentansprüche**

**Patentansprüche für folgende Vertragsstaaten : BE, CH, DE, FR, GB, IT, LI, NL, SE**

1. Primycin enthaltendes kolloidales Grundgel, gekennzeichnet durch einen Gehalt an N-Methyl-pyrrolidon-2 und bis zu 30 Gew.% Primycin.

2. Grundgel nach Anspruch 1, gekennzeichnet durch einen Gehalt an 5-30 Gew.% Primycin und 95-70 Gew.% N-Methyl-pyrrolidon-2, vorzugsweise an 10-20 Gew.% Primycin und 90-80 Gew.% N-Methyl-pyrrolidon-2.

3. Verfahren zur Herstellung eines Primycin enthaltenden kolloidalen Grundgels gemäss Anspruch 2, dadurch gekennzeichnet, daß man 5-30 Gew.% Primycin in 95-70 Gew.% N-Methyl-pyrrolidon-2,vorzugsweise 10-20 Gew.% Primycin in 90-80 Gew.% N-Methyl-pyrrolidon-2,bei einer Temperatur von 50-150 °C, vorzugsweise bei 70-100 °C,gewünschtenfalls unter Rühren, löst und danach die Lösung auf Raumtemperatur abkühlt.

4. Primycin enthaltendes kolloidales Grundgel nach Anspruch 1, gekennzeichnet durch einen zusätzlichen Gehalt an einem oder mehreren antibakteriellen Wirkstoffen, vorzugsweise Antibiotika.

5. Grundgel nach Anspruch 4, dadurch gekennzeichnet, daß es 5-12 Gew.% Primycin, 20-48 Gew.% N-Methyl-pyrrolidon-2, 10-60 Gew.% eines oder mehrerer antibakterieller Wirkstoffe - vorzugsweise Antibiotika - und 15-30 Gew.% Wasser, vorzugsweise 10 Gew.% Primycin, 40 Gew.% N-Methyl-pyrrolidon-2, 20 Gew.% eines Antibiotikums und 30 Gew.% Wasser, enthält.

6. Grundgel nach Anspruch 5, dadurch gekennzeichnet, daß es als Antibiotikum Gentamycin oder Doxycyclin enthält.

7. Verfahren zur Herstellung eines Primycin und weitere Wirkstoffe enthaltenden, kolloidalen Grundgels gemäß Anspruch 5 und 6, dadurch gekennzeichnet, daß man 10-60 Gew.% eines Primycin enthaltenden kolloidalen Grundgels, das 5-30 Gew.% Primycin und 95-70 Gew.% N-Methyl-pyrrolidon-2 enthält, mit 90-40 Gew.% einer 30-70 Gew.%igen wässrigen Lösung eines antibakteriellen Wirkstoffes bei einer Temperatur von 35-40 °C umsetzt und danach auf Raumtemperatur kühlt.

8. Verfahren nach Anspruch 7, dadurch gekennzeichnet, daß man 50 Gew.% eines Primycin enthaltenden kolloidalen Grundgels mit 50 Gew.% einer 40 Gew.%igen wässrigen Lösung eines Antibiotikums bei 40 °C umsetzt.

9. Antibakterielles Präparat, besonders zur Behandlung von Akne vulgaris, dadurch gekennzeichnet, daß es als Wirkstoff 0,1-100 Gew.% eines Primycin enthaltenden kolloidalen Grundgels, gewünschtenfalls weitere pharmazeutische Wirkstoffe und 99,9-0 Gew.% übliche pharmazeutische Füllstoffe, Verdünnungsmittel und Hilfsstoffe enthält.

10. Kombinationspräparat nach Anspruch 9, dadurch gekennzeichnet, daß es als Wirkstoff 1-60 Gew.% eines Primycin enthaltenden kolloidalen Grundgels und in einer Menge von 0,1-40 Gew.% einen oder mehrere weitere pharmazeutische Wirkstoffe, insbesondere Antibiotika, chemotherapeutische Mittel, fungistatische oder fungizide Mittel, steroidartige oder nicht-steroidartige antiphlogistische Mittel, die Epithelbildung fördernde Mittel, lokalanästhetische Mittel und/oder Vitamine enthält.

11. Antibakterielles pharmazeutisches Präparat, insbesondere zur Behandlung von Mastitis, dadurch gekennzeichnet, daß es 0,1-100 Gew.% eines, als Wirkstoff Primycin und einen weiteren antibakteriellen Wirkstoff - vorzugsweise ein Antibiotikum - enthaltendes kolloidales Grundgel und 99,9-0 % übliche pharmazeutische Träger, Füllstoffe und/oder Hilfsstoffe enthält.

**Patentansprüche für folgende Vertragsstaaten : ES, GR, AT**

1. Verfahren zur Herstellung eines Primycin enthaltenden kolloidalen Grundgels, dadurch gekennzeichnet, daß man 5-30 Gew.% Primycin in 95-70 Gew.% N-Methyl-pyrrolidon-2, vorzugsweise 10-20 Gew.% Primycin in 90-80 Gew.% N-Methyl-pyrrolidon-2, bei einer Temperatur von 50-150°C, vorzugsweise bei 70-100°C, gewünschtenfalls unter Rühren, löst und danach die Lösung auf Raumtemperatur abkühlt.

2. Verfahren zur Herstellung eines Primycin und weitere Wirkstoffe enthaltenden, kolloidalen Grundgels, dadurch gekennzeichnet, daß man 10-60 Gew.% eines Primycin enthaltenden kolloidalen Grundgels, das 5-30 Gew.% Primycin und 95-70 Gew.% N-Methyl-pyrrolidon-2 enthält, mit 90-40 Gew.% einer 30-70 Gew.%igen wäßrigen Lösung eines antibakteriellen Wirkstoffes bei einer Temperatur von 35-40°C umsetzt und danach auf Raumtemperatur kühlt.

3. Verfahren nach Anspruch 2, dadurch gekennzeichnet, daß man 50 Gew.% eines Primycin enthaltenden kolloidalen Grundgels mit 50 Gew.% einer 40 Gew.%igen wäßrigen Lösung eines Antibiotikums bei 40°C umsetzt.

12

4. Verfahren nach Anspruch 2, dadurch gekennzeichnet, daß man das Primycin enthaltende kolloidale Grundgel mit der wäßrigen Lösung eines antibakteriellen Wirkstoffs zu einem kolloidalen Grundgel umsetzt, das 5 - 12 Gew.% Primycin, 20 - 48 Gew.% N-Methylpyrrolidon-2, 10 - 60 Gew.% eines oder mehrerer antibakterieller Wirkstoffe und 15 - 30 Gew.% Wasser enthält.

5. Verfahren nach Ansprüchen 2 oder 3, dadurch gekennzeichnet, daß man das Primycin enthaltende kolloidale Grundgel mit der wäßrigen Lösung eines Antibiotikums zu einem kolloidalen Grundgel umsetzt, das 10 Gew.% Primycin, 40 Gew.% N-Methyl-pyrrolidon-2, 20 Gew.% Antibiotikum und 30 Gew.% Wasser enthält.

6. Verfahren nach einem der Ansprüche 2, 3 oder 4, dadurch gekennzeichnet, daß man als Antibiotikum Gentamycin oder Doxycyclin verwendet.

7. Verfahren zur Herstellung eines antibakteriellen Präparates insbesondere zur Behandlung von Akne vulgaris in an sich bekannter Weise, dadurch gekennzeichnet, daß man als Wirkstoff 0,1-100 Gew.% eines Primycin enthaltenden kolloidalen Grundgels und gewünschtenfalls weitere pharmazeutische Wirkstoffe und 99,9-0 Gew.% übliche pharmazeutische Füllstoffe, Verdünnungsmittel und Hilfsstoffe verwendet.

8. Verfahren zur Herstellung eines Kombinationspräparates nach Anspruch 6, dadurch gekennzeichnet, daß man als Wirkstoff 1-60 Gew.% eines Primycin enthaltenden kolloidalen Grundgels und in einer Menge von 0,1-40 Gew.% einen oder mehrere weitere pharmazeutische Wirkstoffe, insbesondere Antibiotika, chemotherapeutische Mittel, fungistatische oder fungizide Mittel, steroidartige oder nichtsteroidartige antiphlogistische Mittel, die Epithelbildung fördernde Mittel, lokalanästhetische Mittel und/oder Vitamine verwendet.

9. Verfahren zur Herstellung eines antibakteriellen pharmazeutischen Präparates, insbesondere zur Behandlung von Mastitis, in an sich bekannter Weise, dadurch gekennzeichnet, daß man 0,1-100 Gew.% eines, als Wirkstoff Primycin und einen weiteren antibakteriellen Wirkstoff - vorzugsweise ein Antibiotikum - enthaltendes kolloidales Grundgel und 99,9-0 Gew.% übliche pharmazeutische Träger Füllstoffe und/oder Hilfsstoffe verwendet.

**Claims**
**Claims for the following Contracting States : BE, CH, DE, FR, GB, IT, LI, NL, SE**

1. Colloidal base gel containing primycin, characterised in that it contains N-methyl-pyrrolid-2-one and up to 30% by weight of primycin.

2. Base gel according to claim 1, characterised in that it contains 5-30% by weight of primycin and 95-70% by weight of N-methyl-pyrrolid-2-one, preferably 10-20% by weight of primycin and 96-80% by weight of N-methylpyrrolid-2-one.

3. Process for preparing a colloidal base gel containing primycin according to claim 2, characterised in that 5-30% by weight of primycin are dissolved in 95-70% by weight of N-methyl-pyrrolid-2-one, preferably 10-20% by weight of primycin are dissolved in 90-80% by weight of N-methyl-pyrrolid-2-one, at a temperature of 50-150° C, preferably at 70-100° C, with stirring if desired, and the solution is then cooled to ambient temperature.

4. Colloidal base gel containing primycin according to claim 1, characterised in that it additionally contains one or more antibacterially active substances, preferably antibiotics.

5. Base gel according to claim 4, characterised in that it contains 5-12% by weight of primycin, 20-48% by weight of N-methyl-pyrrolid-2-one, 10-60% by weight of one or more antibacterially active substances - preferably antibiotics - and 15-30% by weight of water, preferably 10% by weight of primycin, 40% by weight of N-methyl-pyrrolid-2-one, 20% by weight of an antibiotic and 30% by weight of water.

6. Base gel according to claim 5, characterised in that it contains gentamycin or doxycyclin as antibiotic.

7. Process for preparing a colloidal base gel containing primycin and other active substances according to claim 4, 5 or 6, characterised in that 10-60% by weight of a primycin-containing colloidal base gel, containing 5-30% by weight of primycin and 95-70% by weight of N-methyl-pyrrolid-2-one, are reacted with 90-40% by weight of a 30-70% by weight aqueous solution of an antibacterially active substance at a temperature of 35-40° C and then cooled to ambient temperature.

8. Process according to claim 7, characterised in that 50% by weight of a primycin-containing colloidal base gel are reacted with 50% by weight of a 40% by weight aqueous solution of an antibiotic at 40° C.

9. Antibacterial preparation, particularly for treating Acne vulgaris, characterised in that it contains as active substance 0.1-100% by weight of a primycin-containing colloidal base gel, optionally other pharmaceutical active substances and 99.9-0% by weight of conventional pharmaceutical fillers, diluents and excipients.

10. Combined preparation according to claim 9, characterised in that it contains as active substance 1-60% by weight of a primycin-containing colloidal base gel and, in an amount of from 0.1-40% by weight, one or more other pharmaceutical active substances, particularly antibiotics, chemotherapeutic agents, fungistatic or fungicidal agents, steroidal or nonsteroidal antiphlogistic agents, agents which promote epithelial formation, local anaesthetics and/or vitamins.

11. Antibacterial pharmaceutical preparation, more particularly for treating mastitis, characterised in that it contains 0.1-100% by weight of a colloidal base gel containing, as active substances, primycin and another antibacterially active substance, preferably an antibiotic, and 99.9-0% by weight of conventional pharmaceutical carriers, fillers and/or excipients.

**Claims for the following Contracting States : ES, GR, AT**

1. Process for preparing a colloidal base gel containing primycin, characterised in that 5-30% by weight of primycin are dissolved in 95-70% by weight of N-methyl-pyrrolid-2-one, preferably 10-20% by weight of primycin are dissolved in 90-80% by weight of N-methylpyrrolid-2-one, at a temperature of 50-150° C, preferably at 70-100° C, with stirring if desired, and the solution is then cooled to ambient temperature.

2. Process for preparing a colloidal base gel containing primycin and other active substances, characterised in that 10-60% by weight of a primycin-containing colloidal base gel, containing 5-30% by weight of primycin and 95-70% by weight of N-methylpyrrolid-2-one, are reacted with 90-40% by weight of a 30-70% by weight aqueous solution of an antibacterially active substance at a temperature of 35-40° C and then cooled to ambient temperature.

3. Process according to claim 2, characterised in that 50% by weight of a primycin-containing colloidal base gel are reacted with 50% by weight of a 40% by weight aqueous solution of an antibiotic at 40° C.

4. Process according to claim 2, characterised in that the colloidal base gel containing primycin is reacted with an aqueous solution of an antibacterially active substance to form a colloidal base gel which contains 5-12% by weight of primycin, 20-48% by weight of N-methyl-pyrrolid-2-one, 10-60% by weight of one or more antibacterially active substances and 15-30% by weight of water.

5. Process according to claim 2 or 3, characterised in that the primycin-containing colloidal base gel is reacted with the aqueous solution of an antibiotic to form a colloidal base gel which contains 10% by weight of primycin, 40% by weight of N-methyl-pyrrolid-2-one, 20% by weight of antibiotic and 30% by weight of water.

6. Process according to one of claims 2, 3 or 4, characterised in that gentamycin or doxycyclin is used as the antibiotic.

7. Process for preparing an antibacterial preparation, more particularly for the treatment of Acne vulgaris in a manner known per se, characterised in that it uses as active substance 0.1-100% by weight of a primycin-containing colloidal base gel, optionally other pharmaceutical active substances and 99.9-0% by weight of conventional pharmaceutical fillers, diluents and excipients.

8. Process for preparing a combined preparation according to claim 6, characterised in that it uses as active substance 1-60% by weight of a primycin-containing colloidal base gel and, in an amount of from 0.1-40% by weight, one or more other pharmaceutical active substances, particularly antibiotics, chemotherapeutic agents, fungistatic or fungicidal agents, steroidal or non-steroidal antiphlogistic agents, agents which promote epithelial formation, local anaesthetics and/or vitamins.

9. Process for preparing an antibacterial pharmaceutical preparation, more particularly for treating mastitis, in a manner known per se, characterised in that it uses 0.1-100% by weight of a colloidal base gel containing, as active substances, primycin and another antibacterially active substance, preferably an antibiotic, and 99.9-0% by weight of conventional pharmaceutical carriers, fillers and/or excipients.

**Revendications**

**Revendications pour les Etats contractants suivants : BE, CH, DE, FR; GB; IT, LI, NL, SE**

1. Gel de base colloïdal contenant de la primycine, caractérisé en ce qu'il contient de la N-méthyl-pyrrolidone-2 et jusqu'à 30% en poids de primycine.

2. Gel de base selon la revendication 1, caractérisé en ce qu'il contient 5-30% en poids de primycine et 95-70% en poids de N-méthyl-pyrrolidone-2, de préférence 10-20% en poids de primycine et 90-80% en poids de N-méthyl-pyrrolidone-2.

3. Procédé pour la préparation d'un gel de base colloïdal contenant de la primycine selon la revendication 2, caractérisé en ce qu'on dissout 5-30% en poids de primycine dans 95-70% en poids de N-méthyl-pyrrolidone-2, de préférence 10-20% en poids de primycine dans 90-80% en poids de N-méthyl-pyrrolidone-2, à une température de 50-150°C, de préférence à 70-100°C, si on le souhaite sous agitation, et on refroidit ensuite la solution jusqu'à la température ambiante.

4. Gel de base colloïdal contenant de la primycine selon la revendication 1, caractérisé en ce qu'il contient en outre un ou plusieurs agents actifs antibactériens, de préférence des antibiotiques.

5. Gel de base selon la revendication 4, caractérisé en ce qu'il contient 5-12% en poids de primycine, 20-48% en poids de N-méthyl-pyrrolidone-2, 10-60% en poids d'un ou plusieurs agents actifs antibactériens - de préférence des antibiotiques -, et 15-30% en poids d'eau, et préférentiellement 10% en poids de primycine, 40% en poids de N-méthyl-pyrrolidone-2, 20% en poids d'un antibiotique et 30% en poids d'eau.

6. Gel de base selon la revendication 5, caractérisé en ce qu'il contient comme antibiotique de la gentamycine ou de la doxycycline.

7. Procédé pour la préparation d'un gel de base colloïdal contenant de la primycine et d'autres agents actifs selon la revendication 4, 5 ou 6, caractérisé en ce qu'on fait réagir 10-60% en poids d'un gel de base colloïdal contenant de la primycine, qui contient 5-30% en poids de primycine et 95-70% en poids de N-méthyl-pyrrolidone-2, avec 90-40% en poids d'une solution aqueuse à 30-70% en poids d'un agent actif antibactérien à une température de 35-40°C et on refroidit ensuite à la température ambiante.

8. Procédé selon la revendication 7, caractérisé en ce qu'on fait réagir 50% en poids d'un gel de base colloïdal contenant de la primycine avec 50% en poids d'une solution aqueuse à 40% en poids d'un antibiotique, à 40°C.

9. Préparation antibactérienne, en particulier pour le traitement de l'acne vulgaris, caractérisée en ce qu'elle contient comme principe actif 0,1-100% en poids d'un gel de base colloïdal contenant de la primycine et si on le souhaite d'autres principes actifs pharmaceutiques, et 99,9-0% en poids de charges pharmaceutiques, d'agents diluants et d'adjuvants classiques.

10. Préparation combinée selon la revendication 9, caractérisée en ce qu'elle contient comme principe actif 1-60% en poids d'un gel de base colloïdal contenant de la primycine et en une quantité de 0,1-40% en poids un ou plusieurs autres principes actifs pharmaceutiques, en particulier des antibiotiques, des agents de chimiothérapie, des agents fongistatiques ou fongicides, des agents antiphlogistiques de type stéroïdique ou non stéroïdique, des agents accélérant la formation de 1' épithélium, des agents anesthésiques locaux et/ou des vitamines.

11. Préparation pharmaceutique antibactérienne, en particulier pour le traitement de la mastite, caractérisée en ce qu'elle contient 0,1-100% en poids d'un gel de base colloïdal contenant comme principe actif de la primycine et un autre principe actif antibactérien - de préférence un antibiotique - et 99,9-0% en poids de supports, charges et/ou adjuvants pharmaceutiques classiques.

**Revendications pour les Etats contractants suivants : ES, GR, AT**

1. Procédé pour la préparation d'un gel de base colloïdal contenant de la primycine, caractérisé en ce qu'on dissout 5-30% en poids de primycine dans 95-70% en poids de N-méthyl-pyrrolidone-2, de préférence 10-20% en poids de primycine dans 90-80% en poids de N-méthylpyrrolidone-2, à une température de 50-150°C, de préférence à 70-100°C, si on le souhaite sous agitation, et on refroidit ensuite la solution jusqu'à la température ambiante.

2. Procédé pour la préparation d'un gel de base colloïdal contenant de la primycine et d'autres agents actifs, caractérisé en ce qu'on fait réagir à une température de 35-40°C, 10-60% en poids d'un gel de base colloïdal contenant de la primycine, qui contient 5-30% en poids de primycine et 95-70% en poids de N-méthyl-pyrrolidone-2, avec 90-40% en poids d'une solution aqueuse à 30-70% en poids d'un agent actif antibactérien et on refroidit ensuite à la température ambiante.

3. Procédé selon la revendication 2, caractérisé en ce qu'on fait réagir 50% en poids d'un gel de base colloïdal contenant de la primycine avec 50% en poids d'une solution aqueuse à 40% en poids d'un antibiotique, à 40°C.

4. Procédé selon la revendication 2, caractérisé en ce qu'on fait réagir le gel de base colloïdal contenant de la primycine avec la solution aqueuse d'un agent actif antibactérien pour former un gel de base colloïdal qui contient 5-12% en poids de primycine, 20-48% en poids de N-méthyl-pyrrolidone-2, 10-60% en poids d'un ou plusieurs agents actifs antibactériens et 15-30% en poids d'eau.

5. Procédé selon l'une des revendications 2 ou 3, caractérisé en ce qu'on fait réagir le gel de base colloïdal contenant de la primycine avec la solution aqueuse d'un antibiotique pour former un gel de base colloïdal qui contient 10% en poids de primycine, 40% en poids de N-méthyl-pyrrolidone-2, 20% en poids d'un antibiotique et 30% en poids d'eau.

6. Procédé selon l'une quelconque des revendications 2, 3 ou 4, caractérisé en ce qu'on utilise comme antibiotique de la gentamycine ou de la doxycycline.

7. Procédé pour la préparation d'une composition antibactérienne, en particulier pour le traitement de l'acne vulgaris de manière connue en soi, caractérisé en ce qu'on utilise comme principe actif 0,1-100% en poids d'un gel de base colloïdal contenant de la primycine et si on le souhaite d'autres principes actifs pharmaceutiques et 99,9-0% en poids de charges, agents diluants et adjuvants pharmaceutiques classiques.

8. Procédé pour la préparation d'une préparation combinée selon la revendication 6, caractérisé en ce qu'on utilise comme principe actif 1-60% en poids d'un gel de base colloïdal contenant de la primycine et en une quantité de 0,1-40% en poids un ou plusieurs autres principes actifs pharmaceutiques, en particulier des antibiotiques, des agents de chimio-thérapie, des agents fongistatiques ou fongicides, des agents antiphlogistiques de type stéroïdique ou non stéroïdique, des agents accélérant la formation de l'épithélium, des agents anesthésiques locaux et/ou des vitamines.

9. Procédé pour la préparation d'une combinaison pharmaceutique antibactérienne, en particulier pour le traitement de la mastite de manière connue en soi, caractérisé en ce qu'on utilise 0,1-100% en poids d'un gel de base colloïdal contenant comme agent-actif de la primycine et un autre agent actif antibactérien - de préférence un antibiotique - et 99,9-0% en poids de supports, charges et/ou adjuvants pharmaceutiques classiques.